# EUROPÄISCHE PATENTSCHRIFT

(11) **EP 4 004 530 B1**
(45) Veröffentlichungstag und Bekanntmachung des Hinweises auf die Patenterteilung: **15.11.2023**
(21) Anmeldenummer: 20734853.3
(22) Anmeldetag: 17.06.2020
(51) Int. Cl.: G01N 25/52, G01N 33/22

(54) **FLAMMÜBERWACHUNG BEI FLAMMPUNKTBESTIMMUNG ODER BRENNPUNKTBESTIMMUNG**
FLAME MONITORING IN FLASH POINT DETERMINATION OR FIRE POINT DETERMINATION
SURVEILLANCE DE FLAMME DANS LA DÉTERMINATION DU POINT ÉCLAIR OU LA DÉTERMINATION DU POINT DE FEU

(30) Priorität: 30.07.2019 DE 102019120512
(43) Veröffentlichungstag der Anmeldung: 01.06.2022
(73) Patentinhaber: Anton Paar ProveTec GmbH, 15827 Blankenfelde-Mahlow (DE)
(72) Erfinder: STRASSER, Florian, 12207 Berlin (DE); TUAEV, Xenia, 12309 Berlin (DE)
(74) Vertreter: Dilg, Haeusler, Schindelmann Patentanwaltsgesellschaft mbH
(86) Internationale Anmeldenummer: PCT/EP2020/066725
(87) Internationale Veröffentlichungsnummer: WO 2021/018465

(56) Entgegenhaltungen:
- Anonymous: "Herzog Optiflash Pensky Martens Easy, Safe and Accurate Flash Point Determination", , 2015, XP055734028, Gefunden im Internet: URL:https://www.optimus.be/brochures/10400 0.pdf [gefunden am 2020-09-25]
- Anonymous: "SharpEye 20/20R Single IR Flame Detector", , Juli 2006 (2006-07), XP055734058, Gefunden im Internet: URL:https://gastech.com/files/brochures/20 20R-Brochure.pdf [gefunden am 2020-09-25]
- Anonymous: "Tanaka aco-8/aco-8as Automatic Petroleum Tester", , Januar 2012 (2012-01), XP055734064, Gefunden im Internet: URL:https://www.tanaka-sci.com/en/products /pdf/aco-8.pdf [gefunden am 2020-09-25]

## Beschreibung

### Gebiet der Erfindung

Die vorliegende Erfindung betrifft eine Vorrichtung zur Flammpunktbestimmung und/oder Brennpunktbestimmung einer in einem Behälter aufnehmbaren flüssigen Probe und zur Flammüberwachung. Ferner betrifft die vorliegende Erfindung ein Verfahren zur Flammüberwachung bei einer Flammpunktbestimmung und/oder Brennpunktbestimmung einer in einem Behälter aufnehmbaren flüssigen Probe.

### Stand der Technik

Flammpunkt-Prüfapparaturen werden herkömmlicherweise zur Charakterisierung von Brennstoffen (z.B. Diesel, Benzin, Kerosin, Heizöl), Lösemitteln, Schmierölen oder Chemikalien verwendet. Per Definition ist der Flammpunkt die niedrigste Temperatur, bei der sich in einem offenen oder geschlossenen Gefäß oder Tiegel aus der zu prüfenden Flüssigkeit unter festgelegten Bedingungen Dämpfe (gasförmige Probe gemischt mit Luft) in solcher Menge entwickeln, dass sich im oder außerhalb des Behälters ein durch Fremdzündung entflammbares Probengas-Luft-Gemisch bildet.

Zur Bestimmung des Flammpunktes und/oder des Brennpunktes wird, vorzugsweise gemäß verschiedenen Standards, eine definierte Menge einer zu untersuchenden Probe (Substanz) in den Behälter (z.B. Messtiegel) gefüllt, kontrolliert erhitzt (insbesondere auf eine vorbestimmte Temperatur gebracht) und bei Bedarf gerührt. Dabei bildet sich kontinuierlich eine gasförmige Phase über der flüssigen Probe aus. Ab einer bestimmten Temperatur wird in periodischen Zeit- und/oder Temperaturabständen eine Zündquelle in den Behälter eingeführt, um das gebildete Gas-Luft-Probengemisch zu entzünden. Wird bei einer bestimmten Probentemperatur eine Flamme detektiert, deren Brenndauer kleiner als 5 Sekunden ist, so ist der Flammpunkt ermittelt. Ist die Brenndauer länger als 5 Sekunden, so ist der Brennpunkt der Probe bestimmt.

Zur Flammpunktbestimmung eignen sich unterschiedliche Standardmethoden, die im Wesentlichen durch die Methoden nach i) Pensky, ii) Pensky-Martens, iii) Abel, iv) Abel-Pensky, v) Tagliabue und vi) Cleveland charakterisiert sind.

Bei einer Flammpunktbestimmung und/oder Brennpunktbestimmung besteht das Risiko eines Brandes der Probe. Die Probe kann z.B. entzündet werden (selbständig oder durch einen Zünder) und kann unter Flammenbildung dauerhaft verbrennen. Weiterhin kann ein sogenannter "Burn" entstehen, d.h. eine kurzzeitige starke unbeabsichtigte Entflammung, wenn sich die Dampfphase so schnell bildet, dass bei Zündung kurzzeitig eine Flamme außerhalb oder auch innerhalb des Probenraumes entsteht.

Um Sicherheit bei der Gefahr eines Brandes oder eines Burns zu gewährleisten, werden im Stand der Technik Sensoren zur Brandüberwachung, beispielsweise UV- oder Temperatur-Sensoren verwendet. Typischerweise müssen zur Brand-Erkennung oder Burn-Erkennung mehrere Temperatursensoren vorgesehen und platziert werden, um einen großen Überwachungsraum abzudecken, um einen Brand oder einen Burn innerhalb des Probengefäßes oder um das Probengefäß herum zuverlässig und sicher erkennen zu können. Temperatursensoren zur Flammüberwachung bzw. Brandüberwachung und Burnüberwachung haben jedoch bezüglich eines Ansprechverhaltens, einer Antwortzeit, einer Alterung und mechanischen Anfälligkeit Nachteile.

Die Druckschrift DE 103 24 315 A1 offenbart ein Verfahren zur Überwachung der Qualität eines von einem Reformer für den Betrieb von Brennstoffzellen gelieferten Gasgemisches. Dabei wird das Reformat zur Bestimmung der Qualität einem Brenner des Reformers zugeführt und die Zündung des Reformats erfolgt im Brennerraum automatisch. In Abhängigkeit von der dabei gebildeten Flamme wird von einem Sensor ein Messsignal erzeugt, dessen Größe zur Bestimmung der Qualität des Reformats ausgewertet wird. Dabei wird vorzugsweise ein Ionisationsstromsignal von einem Flammenionisationsdetektor zur Bestimmung des Grades der Verunreinigung des Reformats durch Kohlenwasserstoffe ausgewertet.

Die Druckschrift DE 27 23 157 A1 offenbart ein Verfahren zum Ermitteln des Flammpunktes einer zu untersuchenden brennbaren Flüssigkeit, wobei mittels eines Flammenionisationsdetektors (FID) der Gesamtkohlenstoff-Konzentrationswert der zu untersuchenden brennbaren Flüssigkeit gemessen wird.

Die Druckschrift DE 197 15 757 A1 offenbart ein Verfahren zum Betrieb von Kohlebrennern, wobei aus zur Verbrennung bestimmter Kohle durch Laserstrahlbeschuss ein Plasma gebildet wird, das einer Spektralanalyse unterworfen wird, um so die Bestandteile der Kohle zu ermitteln.

Die Druckschrift US 2014/0326049 A1 offenbart ein Verfahren zur Echtzeitmessung von Treibstoffgaskompositionen und Heizwerten, wobei der Messapparat einen Nah-Infrarot-Sensor zum Messen von Konzentrationen von Kohlenwasserstoffen und Kohlendioxid umfasst. Eine Verbrennung wird nicht durchgeführt.

Die Druckschrift US 5,822,058 A offenbart Verfahren zum optischen Messen von Eigenschaften von Kohlenwasserstoff-Treibstoffgasen. Eine Lichtquelle erzeugt Licht bei nah-sichtbaren Wellenlängen und wird durch die Probe transmittiert. Ein Spektrometer dispergiert das die Probe durchsetzende Licht und ein spektrales Bild wird aufgenommen und verarbeitet.

Das Dokument DE 101 21 641 A1 offenbart ein Verfahren und eine Vorrichtung zum Ermitteln der Gasbeschaffenheit eines Erdgases, wobei das Brenngas einer Infrarotbestrahlung ausgesetzt wird und wobei für zwei Wellenlängen oder spektrale Bereiche jeweils der vom Brenngas absorbierte Anteil der Infrarotstrahlung erfasst wird. Daraus wird die Gasbeschaffenheit bestimmt.

Die Druckschrift US 4,845,040 A offenbart ein Verfahren zum Analysieren verschiedener Schwefelformen, welche z.B. in Kohle vorhanden sind. Eine die Probe enthaltende Mischung wird innerhalb einer Verbrennungszone verbrannt und die Verbrennungsgase werden durch einen Infrarotanalysator geführt, welcher kontinuierlich die Intensität der Infrarotspektren überwacht. Aus den verschiedenen Infrarotintensitätsmustern kann auf die verschiedenen Formen von Schwefel zurückgeschlossen werden.

Die Druckschrift CA 1 316 800 C offenbart ein Verfahren zum Analysieren verschiedener Schwefelformen ähnlich wie in dem voran beschriebenen Dokument offenbart ist.

Die Druckschrift DE 196 50 302 A1 offenbart ein Verfahren sowie eine Vorrichtung zur Bestimmung der Gasbeschaffenheit einer Gasmischung, wobei die Gasmischung einer Infrarotstrahlung ausgesetzt wird und der von der Gasmischung absorbierte Anteil der Infrarotstrahlung gemessen wird, woraus die Methanzahl der Gasmischung bestimmt wird.

Der Flammpunkprüfer aco-8 von Tanaka-Scientific verwendet einen lonisationsdetektor.

In konventionellen Flammpunktprüfern wird die Brand-und Burnerkennung durch UV-Licht-oder Temperatursensoren durchgeführt. Beide Überwachungsarten sind zwar grundsätzlich für die Erkennung von Flammen geeignet, haben jedoch beispielsweise folgende Schwächen:
- Die Verlässlichkeit der Überwachung mit UV-Licht-Sensoren ist von den Umgebungsbedingungen abhängig. Wenn die Positionierung des Messgerätes ungünstig gewählt wird und die Messung unter Einstrahlung von Licht mit hohem UV-Anteil durchgeführt wird, kann die Verlässlichkeit beeinträchtigt werden. Ebenso verhindert der Einsatz von bestimmten UV-Licht-filternden Materialien (Fensterglas z.B.) eine verlässliche Detektion. Da nicht jede Verbrennung gleich viel UV-Licht erzeugt, kann die Detektion von manchen Proben zu Problemen führen.
- Der Einsatz von Temperatursensoren kann durch mehrere Faktoren suboptimal sein: i) schwankende/hohe Umgebungswärme kann zu Störungen führen, ii) Ansprechgeschwindigkeit und Sensibilität ist durch die Bauart beeinträchtigt: materialspezifische Wärmeleitkoeffizienten und -widerstände begrenzen die Reaktionszeit, iii) Notwendigkeit des Einsatzes mehrerer Sensoren, um die notwendige Detektionsfläche abzudecken und iv) Fehlsignale können durch benachbarte heiße Teile des Messgerätes entstehen (Zündung ca. 1300°C, Tiegeldeckel bzw. Probentemperatur bis zu 405°C). Es müssen mehrere Sensoren an unterschiedlichen Orten platziert werden, um eine Gaszündung von einem Brand zu unterscheiden. Diese Sensoren müssen getrennt voneinander ausgelesen werden, um einen Brand sicher zu erkennen, was die Detektion aufwendig macht.

Es ist somit eine Aufgabe der vorliegenden Erfindung, eine Vorrichtung bzw. ein Verfahren zur Flammüberwachung im Zusammenhang mit einer Flammpunktbestimmung und/oder Brennpunktbestimmung zu schaffen, wobei eine Brand-Erkennung und/oder eine Burn-Erkennung zuverlässig mit schneller Ansprechzeit und hoher Sicherheit ermöglicht ist. Als ein Nebenaspekt ist es eine weitere Aufgabe der vorliegenden Erfindung, ein Verfahren und eine Vorrichtung anzugeben, um sicher und zuverlässig das Vorhandensein einer Flamme eines Gaszünders zu überwachen.

### Zusammenfassung der Erfindung

Die Aufgabe wird durch die Gegenstände der unabhängigen Ansprüche gelöst, die auf eine Vorrichtung bzw. ein Verfahren gerichtet sind. Die abhängigen Ansprüche spezifizieren besondere Ausführungsformen der vorliegenden Erfindung.

Gemäß einer Ausführungsform der vorliegenden Erfindung ist eine Vorrichtung zur Flammpunktbestimmung und/oder Brennpunktbestimmung einer in einem Behälter aufnehmbaren flüssigen Probe und zur Flammenerkennung bereitgestellt, aufweisend eine Behälteraufnahme zum Aufnehmen des Behälters; einen Infrarot-Sensor, der angeordnet ist, Licht, das durch eine Flamme in einem Bereich um oder innerhalb des Behälters erzeugt ist, zu detektieren; und ein Auswertesystem, das mit dem Infrarot-Sensor gekoppelt ist und ausgebildet ist, Signale des Infrarot-Sensors auszuwerten, um basierend auf der Auswertung einen Brand und/oder einen Burn zu unterscheiden/zu erkennen.

Per Definition ist der Flammpunkt die niedrigste Temperatur, bei der sich in einem offenen oder geschlossenen Tiegel aus der zu prüfenden Flüssigkeit unter festgelegten Bedingungen Dämpfe in solcher Menge entwickeln, dass sich im oder außerhalb des Tiegels ein durch Fremdzündung entflammbares Dampf-Luft-Gemisch bildet.

Zur Bestimmung des Flamm-und/oder Brennpunktes wird eine definierte Menge einer zu untersuchenden Substanz in einen Behälter (z.B. Messtiegel) gefüllt, kontrolliert erhitzt und bei Bedarf gerührt, wobei sich kontinuierlich ein Flüssig-Dampf-Gemisch der Probe ausbildet. In bestimmten Abständen der Probentemperaturveränderung wird eine Zündquelle in den Tiegel eingeführt, um das gebildete Flüssig-Dampf-Gemisch zu zünden. Wird eine Flamme, deren Brenndauer < 5 s ist, detektiert, so ist der Flammpunkt ermittelt. Ist die Brenndauer länger als 5 s, so ist der Brennpunkt der Probe bestimmt.

Die Vorrichtung kann geeignet sein für einen standardisierten Flammpunktbestimmungstest und/oder Brennpunktbestimmungstest, welcher z.B. einer oder mehreren der folgenden Normen entspricht (jeweils zumindest für die am Anmeldungsdatum gültigen Versionen): ASTM D93, DIN EN ISO 2719, GB/T261, IP 34, JIS K 2265, ISO 13736, ISO 1516, ISO 1523, DIN 51755-1 (Abel-Pensky mit entsprechendem Zubehör); ASTM D56, ASTM D3934, ASTM D3941; ASTM D92, DIN EN ISO 2592, IP 36, IP 403. Ausführungsformen können weiteren hier nicht aufgeführten Normen entsprechen. Ausführungsformen der vorliegenden Erfindung unterstützten eine oder mehrere der Methoden nach i) Pensky und/oder ii) Pensky-Martens und/oder iii) Abel und/oder iv) Abel-Pensky und/oder v) Tagliabue und/oder vi) Cleveland. Dabei können die Vorrichtungen gemäß Ausführungsformen der vorliegenden Erfindung den folgenden Normen entsprechen: ASTM D93, EN ISO 2719, GB/T261, IP 34, JIS K2265; ASTM D92, EN ISO 2592, IP 36, IP 403, JIS K2265 (jeweils zumindest für die am Anmeldungsdatum gültigen Versionen).

Zur Flammpunktbestimmung eignen sich unterschiedliche durch Ausführungsformen der vorliegenden Erfindung unterstützte Standardmethoden, die im Wesentlichen durch die Methoden nach i) Pensky, ii) Pensky-Martens, iii) Abel, iv) Abel-Pensky, v) Tagliabue und vi) Cleveland charakterisiert sind. Z.B. werden durch Ausführungsformen der vorliegenden Erfindung folgende Normen für Flammpunktprüfer unterstützt: ASTM D93, DIN EN ISO 2719, GBjT261, IP 34, JIS K 2265; ISO 13736, ISO 1516, ISO 1523, DIN 51755-1 (Abel Pensky mit entsprechendem Zubehör); ASTM D56, ASTM D3934 (Blitz/kein Blitz), ASTM D3941 (Gleichgewichtsverfahren); ASTM D92, EN ISO 2592, IP 36, IP 403.

Das Risiko eines Brandes der Probe ist während einer Flammpunktmessung stets vorhanden. Als Ursachen sind z.B. Falschbedienung bzw. unbekannte Probenzusammensetzung denkbar. Um die Sicherheit des Bedieners zu gewährleisten, wird ein Flammpunktmessgerät mit zumindest einem Infrarot-Sensor (und insbesondere auch mit einem FID-Sensor, s.u.) zur Brandüberwachung ausgestattet. Damit kann auch ein so genannter Burn rechtzeitig erkannt werden. Als Burn wird das Phänomen bezeichnet, wenn während der Probenerhitzung im Flammpunktmessgerät die Dampfphase sich derart schnell bildet, dass bei einem Entzündungsversuch ungeplante kurzzeitige Entflammung außerhalb (und/oder teilweise innerhalb) des Probenraumes (d.h. Behälters) oder um den Probenraum herum stattfindet. Solche Verpuffung der Probe muss detektiert werden, da die Richtigkeit der darauf folgenden Flammpunkterkennung nicht mehr gewährleitet werden kann. In Folge muss die Messung abgebrochen und mit frischer Probe neu gestartet werden.

Der Behälter kann z.B. eine Zylinderform aufweisen. Während der Flammpunktbestimmung und/oder Brennpunktbestimmung kann der Behälter teilweise z.B. zu einem Drittel bis zur Hälfte mit der flüssigen Probe befüllt sein. Bei der Probe kann es sich z.B. um einen Brennstoff, um ein Lösungsmittel, ein Schmieröl oder andere Chemikalien handeln. Die Probe kann eine Mischung oder eine Reinsubstanz sein. Die Behälteraufnahme kann eine Temperiervorrichtung umfassen, um den Behälter und somit auch die darin aufgenommene Probe während der Flammpunktbestimmung und/oder Brennpunktbestimmung auf eine gewünschte definierte Temperatur zu temperieren, insbesondere aufzuheizen. Die Behälteraufnahme kann z.B. als ein Heizblock ausgeführt sein, welcher aus Metall gefertigt ist und eine elektrische Heizung insbesondere Heizdrähte aufweist.

Der Infrarot-Sensor kann in einer festen oder variablen räumlichen Beziehung zu der Behälteraufnahme installiert sein. Der Infrarot-Sensor kann derart positioniert sein, dass von einem gewünschten Überwachungsbereich ausgehende Infrarotstrahlung durch den Infrarot-Sensor detektierbar ist. Der Überwachungsbereich kann einen Bereich innerhalb und/oder um den Behälter herum umfassen.

Bei der Flamme kann es sich um eine kurzzeitige Flamme (z.B. bei einem Burn) oder um eine kontinuierliche Flamme (z.B. bei einem Brand) handeln. Der Infrarot-Sensor kann gemäß einer Ausführungsform der vorliegenden Erfindung ausgebildet sein, sowohl Wärmestrahlung zu detektieren als auch das Vorhandensein von Kohlendioxid zu detektieren, indem z.B. Wärmestrahlung innerhalb eines bestimmten Spektralbereiches detektiert wird. Wenn sowohl das Vorhandensein einer Wärmestrahlung als solches detektierbar ist als auch detektierbar ist, ob bzw. wieviel Kohlendioxid vorhanden ist, kann zuverlässig von dem Infrarot-Sensor detektiert werden, ob eine Flamme (z.B. eines Burns oder eines Brandes) in dem Überwachungsraum vorhanden ist. Der Infrarot-Sensor kann kontinuierlich in zeitlich aufeinanderfolgenden Intervallen die Infrarot-Sensor-Daten aufnehmen (z.B. für eine Differentialmessung), um daraus z.B. auch den Zeitraum bestimmen zu können, über den die Flamme vorhanden ist.

Gemäß Ausführungsformen der vorliegenden Erfindung kann der Infrarot-Sensor ortsauflösend sein. Somit kann z.B. der Infrarot-Sensor die gesamte Infrarotstrahlung, die aus dem Überwachungsbereich ausgeht, aufnehmen. Damit kann die Empfindlichkeit des Infrarot-Sensors vorteilhaft erhöht sein. Der Infrarot-Sensor kann die Flammüberwachung während einer Vorbereitung und/oder einer Durchführung und/oder Nachbereitung einer Flammpunktbestimmung und/oder Brennpunktbestimmung kontinuierlich durchführen, insbesondere bei geöffnetem oder geschlossenem Behälter.

Beim Temperieren der Probe bzw. Aufheizen der Probe kann ein Behälter typischerweise durch einen Behälterdeckel verschlossen sein. Falls die Probe z.B. fälschlicherweise zu stark aufgeheizt wurde, kann sich die Probe bei geschlossenem Behälter spontan selbständig oder durch Fremdzündung entzünden, was zu einem "Burn" (kann ähnlich einer Stichflamme sein) führen kann. Ein solcher Burn kann auch durch Teile der Probe entstehen, welche unbeabsichtigt auf Teilen der Vorrichtung um den Behälter bzw. um die Behälteraufnahme herum verschüttet worden ist. Ein Burn kann somit innerhalb und/oder außerhalb des Behälters z.B. bei geschlossenem Behälter und auch bei geöffnetem Behälter entstehen, was durch den Infrarot-Sensor überwacht werden kann.

Ist eine gewünschte Temperatur der Probe erreicht, so kann der Behälterdeckel geöffnet oder von dem Behälter entfernt werden, um so bei geöffnetem Behälter eine Zündvorrichtung in den Innenraum des Behälters einführen zu können, um einen Zündversuch der Probe durchzuführen. Auch hierbei kann entweder ein Burn oder gar ein Brand entstehen, wobei der Brand mit einer zeitlich anhaltenden Flamme assoziiert ist. Auch ein Brand und/oder ein Burn bei geöffnetem Behälter kann von dem Infrarot-Sensor überwacht und detektiert werden. Ein Brand oder ein Burn kann aufgrund der Infrarot-Sensor-Daten z.B. mittels des Auswertesystems angezeigt werden, falls die Infrarot-Sensor-Daten einen oder mehrere Schwellwerte für eine oder mehrere Zeiträume übersteigen.

Vorteilhafterweise kann der Infrarot-Sensor räumlich entfernt von dem Überwachungsbereich angeordnet sein, um somit nicht unnötigerweise Raum nahe dem Messraum einzunehmen und auch um eine Beschädigung des Infrarot-Sensors z.B. durch Probengas oder die Flamme zu reduzieren oder gar zu vermeiden. Ferner kann der Infrarot-Sensor eine schnellere Ansprechzeit aufweisen als herkömmlich verwendete Temperatursensoren.

Gemäß einer Ausführungsform der vorliegenden Erfindung kann der Infrarot-Sensor einen für Wärmestrahlung empfindlichen Bereich sowie zumindest ein optisches Filter aufweisen, das ausgebildet ist und angeordnet ist, um von dem für Wärmestrahlung empfindlichen Bereich zumindest einen Teil zumindest einer Absorptionsbande von Kohlendioxid zu detektieren.

Das optische Filter kann z.B. Wellenlängen, welche außerhalb der zumindest einen (oder mehrerer) Absorptionsbande(n) von Kohlendioxid liegen wesentlich abschwächen (z.B. um mehr als 80% oder mehr als 90%) relativ zu Wellenlängen, die innerhalb der (einen oder der mehreren) Absorptionsbande(n) von Kohlendioxid liegen. Bei jeder Verbrennung eines organischen Brennstoffes, welcher (per Definition) Kohlenstoff enthält, entsteht zum Teil Kohlendioxid. Wenn somit die von dem Infrarot-Sensor detektierten Infrarot-Sensor-Daten auch eine (relative) oder differentielle Konzentration von Kohlendioxid anzeigen, so kann geschlossen werden, dass die von dem Infrarot-Sensor detektierten Daten tatsächlich aufgrund einer Verbrennung herrühren. Somit kann der Infrarot-Sensor heiße Bereiche des Überwachungsbereiches dahingehend differenzieren, ob mehr oder weniger Kohlendioxid vorhanden ist.

Innerhalb des Überwachungsbereiches kann auch der Behälter sowie möglicherweise Teile der Behälteraufnahme liegen. Diese Teile der Behälteraufnahme bzw. der Behälter selbst werden bei einer normalen Durchführung einer Flammpunktbestimmung bzw. Brennpunktbestimmung erwärmt, erzeugen jedoch bei der Erwärmung kein Kohlendioxid. Eine Aufheizung der Behälteraufnahme bzw. des Behälters kann aufgrund des optischen Filters von einer Wärmestrahlung, die aufgrund einer Verbrennung entsteht, unterschieden werden. Damit kann die Spezifität der Flammüberwachung bzw. die Zuverlässigkeit der Flammüberwachung erhöht werden, insbesondere kann die Zahl von falschen Flamm- bzw. Brand- bzw. Burn-Anzeigen vermindert werden.

Gemäß einer Ausführungsform der vorliegenden Erfindung liegt die Absorptionsbande in einem Wellenlängenbereich zwischen 4,2 µm und 4,4 µm. In anderen Ausführungsformen kann eine noch weitere Absorptionsbande von Kohlendioxid ausgebildet werden, z.B. in einem Bereich zwischen 13 und 15 µm. In dem Wellenlängenbereich zwischen 4,2 µm und 4,4 µm weist Kohlendioxid eine ausgeprägte Absorptionsbande auf, in welchem Bereich andere Luftbestandteile oder Gasbestandteile keine signifikante Absorptionsbande aufweisen. Damit kann die Spezifität des Infrarot-Sensors zum Nachweis der Anwesenheit von Kohlendioxid verbessert werden, wodurch die Zuverlässigkeit der Flammüberwachung gesteigert werden kann.

Gemäß einer Ausführungsform der vorliegenden Erfindung weist der Infrarot-Sensor ein oder mehrere Thermoelemente auf, die den für Wärmestrahlung empfindlichen Bereich definieren, und ist insbesondere als eine Thermosäule ausgebildet. Eine Thermosäule oder Thermokette (englisch: *thermopile*) kann als ein elektrisches Bauelement gebildet sein, das thermische Energie (insbesondere Wärmestrahlung) in elektrische Energie verwandelt. Die Thermosäule kann aus mehreren Thermoelementen bestehen, die thermisch parallel und elektrisch in Reihe geschaltet sind, wodurch die sehr geringen Thermospannungen addiert werden. Die Wärmestrahlung kann auf die in Reihe geschalteten Thermoelemente auftreffen, wodurch sich die Spannung zwischen einem Ende der Thermokette und einem anderen Ende der Thermokette ändern kann. Damit können Ausführungsformen der vorliegenden Erfindung durch herkömmlich erhältliche Sensoren implementiert werden.

Ein Abgleich des Infrarot-Sensors muss nicht notwendig sein. Auf einen Brand oder einen Burn kann aufgrund einer differentiellen Messung geschlossen werden. Dabei kann die Änderung der Infrarot-Sensor-Daten herangezogen werden. Ändern sich z.B. die Infrarot-Sensor-Daten plötzlich um einen Betrag höher als ein definierter Schwellwert, so kann auf eine Entflammung geschlossen werden. Der Zeitverlauf des Infrarot-Sensor-Signals kann dann Aufschluss darüber geben, ob ein Burn oder ein Brand vorliegt.

Gemäß der vorliegenden Erfindung weist die Vorrichtung ferner auf einen Flammenionisationsdetektor mit zwei Elektroden, einer Spannungsquelle, um an eine Elektrode eine elektrische Spannung anzulegen, und einer abgleichbaren Widerstandsmessschaltung, insbesondere Wheatstone'sche Messbrücke, um Widerstands-Daten zu messen, die einen Widerstand zwischen den Elektroden abhängig von der Ionisation der Luft, die durch Entflammung entsteht, anzeigen.

Bei einer Flammenbildung können Luftmoleküle aufgrund der thermischen Energie ionisiert werden, wodurch Elektronen und/oder Ionen freigesetzt werden. Die Menge bzw. die Anzahl der Elektronen und/oder Ionen kann eine Stärke der Flamme anzeigen. In Abhängigkeit der Menge und/oder Konzentration der entstandenen Elektronen und /oder Ionen ändert sich der elektrische Widerstand der Luftumgebung (Luft und dampfförmige Phase der Probe) zwischen den zwei Elektroden. Wenn der Widerstand durch die Widerstandsmessschaltung gemessen wird, kann somit auf das Vorhandensein bzw. die Konzentration von Elektronen und/oder Ionen zwischen den Elektroden und somit auf eine Flammenionisation zurückgeschlossen werden.

Die zwei Elektroden können derart angeordnet sein, dass zwischen ihnen ein Bereich liegt, welcher durch Flammenionisationsdetektion zu überwachen ist. Der durch Flammenionisationsdetektion zu überwachende Bereich kann verschieden sein, insbesondere kleiner sein, als der durch den Infrarot-Sensor zu überwachende Bereich. Die zwei Elektroden können derart angeordnet sein, dass zumindest ein Bereich oberhalb der Behälteraufnahme bzw. oberhalb des Behälters durch Flammenionisationsdetektion überwacht werden kann. Dieser Überwachungsbereich kann z.B. in einem Raumbereich von innerhalb des Behälters bzw. von einem oberen Rand des Behälters bis z.B. zwischen 5 mm und 10 mm oberhalb des oberen Randes des Behälters (oder der Behälteraufnahme) liegen.

An die Formen der zwei Elektroden sind keine besonderen Einschränkungen erforderlich, sie müssen nur derart lokalisiert sein, dass der von ihnen zu überwachende Bereich zwischen ihnen liegt. Vorteilhaft kann eine der Elektroden geerdet sein und an die andere kann eine geringe elektrische Spannung angelegt werden, z.B. zwischen 1 Volt und 10 Volt. Eine oder beide der Elektroden können durch Teile eines Gehäuses der Vorrichtung und/oder durch Teile des Behälters bzw. eines Behälterdeckels gebildet sein.

Gemäß einer Ausführungsform der vorliegenden Erfindung ist das Auswertesystem mit dem Flammenionisationsdetektor gekoppelt und ausgebildet, die Widerstands-Daten auszuwerten, wobei basierend auf einem Vergleich einer gemessenen Änderung des Widerstands mit einem Schwellwert auf einen Brand oder Burn und/oder eine Gaszündflamme geschlossen wird.

Die Wheatstone'sche Messbrücke kann den Flammenionisationsdetektor (insbesondere die beiden Elektroden) enthalten. Die Widerstandsbrücke (Wheatstone'sche Messbrücke) ist eine Schaltung aus zwei parallel geschalteten Spannungsteilern. Die Spannung zwischen Abgriffen der Spannungsteiler wird über ein Spannungsmessgerät gemessen. Wenn das Verhältnis der Spannungsteiler-Widerstände gleich groß ist, haben beide Abgriffe zwischen den Spannungsteilern gleiches Potential. Ein Widerstand eines Spannungsteilers kann als ein veränderlicher Widerstand ausgebildet sein, um einen Abgleich einerseits und auch eine Messung nach Abgleich durchführen zu können. Die beiden Elektroden können als ein Widerstand eines Spannungsteilers (oder parallel geschaltet zu einem anderen Widerstand) geschaltet sein.

Ein Abgleich (bei Fehlen jeglicher Flamme in dem Überwachungsbereich) kann vor jeder beabsichtigten Flammpunktbestimmung und/oder Brennpunktbestimmung durchgeführt werden. Bei Flammenbildung zwischen den Elektroden nimmt der Widerstand zwischen den beiden Elektroden ab. Der veränderbare Widerstand der Widerstandsbrücke kann sodann nachgestellt werden, um wiederum eine Spannung von 0 V zwischen den Abgriffen der beiden Spannungsteiler einzustellen oder der Ausschlag von dem auf Null kalibrierten Punkt wird ausgewertet (Ausschlagsverfahren). Die Veränderung des veränderbaren Widerstandes ist dann indikativ für den Grad der Ionisation des Gases zwischen den Elektroden bzw. den Widerstand zwischen den Elektroden und lässt auf eine Flammbildung schließen. Damit ist ein zusätzlicher unabhängiger Sensor zur Flammüberwachung bereitgestellt. Die Infrarot-Sensor-Daten und/oder die Widerstands-Daten können jeweils separat oder in Kombination zur Flammüberwachung herangezogen werden. Die Verrechnungslogik der Infrarot-Sensor-Daten und Widerstands-Daten kann je nach Anwendungsfall variieren.

Gemäß einer Ausführungsform der vorliegenden Erfindung ist das Auswertesystem ausgebildet, eine Gasflammenerkennung eines Gaszünders und/oder eine Burn-Erkennung und/oder eine Brand-Erkennung durchzuführen, wobei für die Brand-Erkennung und/oder die Burn-Erkennung sowohl die Widerstands-Daten als auch die Infrarot-Sensor-Daten herangezogen werden, wobei für die Gasflammenerkennung vorwiegend die Widerstands-Daten herangezogen werden.

Die Gasflammenerkennung des Gaszünders kann während eines normalen Betriebs während einer Flammpunktbestimmung und/oder Brennpunktbestimmung durchgeführt werden. Dabei kann eine Zündspitze des Gaszünders nahe bei den Elektroden, insbesondere teilweise zwischen den Elektroden liegen. Ein Burn kann eine kurzzeitige starke Entflammung (teilweise) innerhalb des Behälters und/oder außerhalb des Behälters umfassen. Ein Brand kann eine länger anhaltende Flamme innerhalb des Behälters oder auch außerhalb des Behälters umfassen. Wenn sowohl die Widerstands-Daten als auch die Infrarot-Sensor-Daten herangezogen werden, um eine Brand-Erkennung und/oder eine Burn-Erkennung zu ermöglichen, kann die Zuverlässigkeit erhöht werden, insbesondere kann die Anzahl falscher Brand-Erkennungen oder Burn-Erkennungen vermindert werden.

Gemäß einer Ausführungsform der vorliegenden Erfindung wird ein Brand erkannt, wenn sowohl die Widerstands-Daten als auch die Infrarot-Sensor-Daten eine Erhöhung über jeweilige Schwellwerte über einen Mindestzeitraum anzeigen. Dabei können jeweils differentielle Messungen herangezogen werden.

Gemäß einer Ausführungsform der vorliegenden Erfindung wird ein Burn erkannt, wenn sowohl die Widerstands-Daten als auch die Infrarot-Sensor-Daten eine Erhöhung über jeweilige Schwellwerte für weniger als 1/5 oder 1/10 der Mindestzeit für einen Brand zeigen. Die Auswertelogik kann je nach Anwendung geändert und auch hinsichtlich der Parameter (Schwellwerte, Mindestzeitraum, etc.) angepasst werden.

Gemäß einer Ausführungsform der vorliegenden Erfindung weist die Vorrichtung ferner einen Behälter zum Aufnehmen der flüssigen Probe mit einer Behälteröffnung auf. Der Behälter kann aus Metall gefertigt und zylinderförmig sein. Der Behälter ist aus der Behälteraufnahme herausnehmbar. Die Behälteröffnung kann kreisförmig sein. Somit werden herkömmliche normgerechte Flammpunkt- und/oder Brennpunktbestimmungen unterstützt.

Gemäß einer Ausführungsform der vorliegenden Erfindung ist der Infrarot-Sensor oberhalb der Behälteröffnung angeordnet, insbesondere in einem Bereich zwischen 5 cm und 30 cm von einer oberen Kante des Behälters entfernt. Somit kann der Infrarot-Sensor vor Beschädigung während der Messung aufgrund der Entfernung geschützt sein und muss ferner nicht unnötigen Raum einnehmen, welcher für andere Messausstattung oder welche zum Hantieren benötigt wird.

In einer Ausführungsform der vorliegenden Erfindung weist die Vorrichtung ferner einen Behälterdeckel auf, wobei eine erste der Elektroden durch den Behälter und/oder den Behälterdeckel gebildet ist und wobei die Spannungsquelle ausgebildet ist, die erste Elektrode auf Massepotential zu legen. Damit kann der Behälter bzw. der Behälterdeckel jeweils eine doppelte Funktion ausführen, um auch bei der Flammionisationsdetektion mitzuwirken. Wenn die erste Elektrode auf Massepotential gelegt wird, kann eine Gefahr für einen Benutzer ausgeschlossen werden.

Gemäß einer Ausführungsform der vorliegenden Erfindung ist eine zweite der Elektroden durch ein Metallteil, insbesondere in der Umgebung einer Zündvorrichtung mit zumindest einer Zündspitze, gebildet, die zum Entzünden der Probe in den Behälter hinein, insbesondere geradlinig, verfahrbar ist, wobei die Spannungsquelle ausgebildet ist, die zweite Elektrode auf ein positives elektrisches Potential, insbesondere zwischen 1 V und 10 V, zu legen. Vorteilhafterweise muss somit für die zweite Elektrode nicht ein zusätzliches oder separates leitfähiges Element vorgesehen sein. Damit kann die Vorrichtung vereinfacht werden. Der Teil der Zündvorrichtung kann z.B. ein Teil eines Gehäuses oder einer Ummantelung sein. Die Zündvorrichtung ist vorgesehen, um die (dampfförmige) Probe innerhalb des Behälters zu zünden, wozu sie in den Behälter hinein verfahren wird. Beim Aufheizen der Probe auf einen nächsten Temperaturwert, kann die Zündspitze außerhalb des Behälters verfahren werden und der Behälterdeckel kann auf die Behälteröffnung aufgelegt sein.

Gemäß einer Ausführungsform der vorliegenden Erfindung weist die Zündvorrichtung einen Elektrozünder und/oder einen, insbesondere, abnehmbaren Gaszünder auf. Je nach Norm oder standardisiertem Test, kann eine Elektrozündung und/oder eine Gaszündung erforderlich sein. Damit werden verschiedene Normen oder standardisierte Tests unterstützt. Wenn der Gaszünder abnehmbar ist, kann er entfernt werden, sofern eine Gaszündung nicht für den durchzuführenden standardisierten Test erforderlich ist. Damit kann eine Handhabbarkeit der Vorrichtung verbessert werden.

Gemäß einer Ausführungsform der vorliegenden Erfindung weist die Vorrichtung ferner ein Temperaturmesssystem auf, das ausgebildet ist, eine Temperatur der Probe im flüssigen und/oder gasförmigen Zustand innerhalb des Behälters zu messen, um einen Flammpunkt und/oder einen Brennpunkt zu bestimmen; und weist ferner ein Temperier-System zum Temperieren der Probe auf.

Das Temperier-System kann insbesondere eine elektrische Heizung umfassen, welche z.B. in der Behälteraufnahme vorgesehen ist und wobei die Behälteraufnahme z.B. als ein Temperierblock oder Heizblock ausgeführt sein kann. Somit ist die Vorrichtung ausgebildet, sowohl benutzerdefinierte als auch standardisierte Tests zur Flammpunktbestimmung und/oder Brennpunktbestimmung durchzuführen.

Es sollte verstanden werden, dass Merkmale, welche individuell oder in irgendeiner Kombination im Zusammenhang mit einer Vorrichtung zur Flammpunktbestimmung und/oder Brennpunktbestimmung und zur Flammüberwachung beschrieben, offenbart oder angewendet wurden, ebenso, individuell oder in irgendeiner Kombination, auf ein Verfahren zur Flammüberwachung bei einer Flammpunktbestimmung und/oder Brennpunktbestimmung anwendbar sind, gemäß Ausführungsformen der vorliegenden Erfindung, und umgekehrt.

Gemäß einer Ausführungsform der vorliegenden Erfindung ist ein Verfahren zur Flammüberwachung bei einer Flammpunktbestimmung und/oder Brennpunktbestimmung einer in einem Behälter aufnehmbaren flüssigen Probe bereitgestellt, wie es in Anpruch 15 beschrieben ist.

Ausführungsformen der Erfindung werden nun mit Bezug auf die angehängten Zeichnungen beschrieben. Die Ausführungsformen sind exemplarisch und begrenzen nicht den Geltungsbereich der Erfindung.

### Kurze Beschreibung der Zeichnungen

Fig. 1 illustriert in schematischer Darstellung eine Vorrichtung zur Flammpunktbestimmung und/oder Brennpunktbestimmung und zur Flammüberwachung gemäß einer Ausführungsform der vorliegenden Erfindung;
Fig. 2 illustriert schematisch ein Auswertesystem einer Vorrichtung zur Flammpunktbestimmung und/oder Brennpunktbestimmung und zur Flammüberwachung gemäß einer Ausführungsform der vorliegenden Erfindung; und
Fig. 3 illustriert ein Transmissionsspektrum von Kohlendioxid, wie es von Ausführungsformen der vorliegenden Erfindung berücksichtigt wird.

### Detaillierte Beschreibung von Ausführungsformen

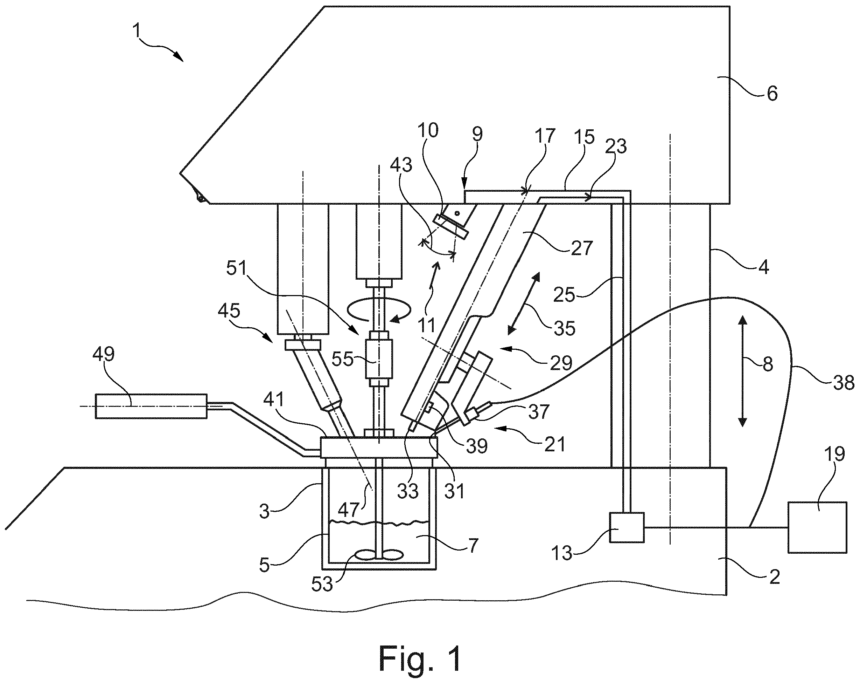

Die in Fig. 1 schematisch in einer Seitenschnittansicht illustrierte Vorrichtung 1 zur Flammpunktbestimmung und/oder Brennpunktbestimmung einer in einem Behälter aufnehmbaren flüssigen Probe und zur Flammüberwachung gemäß einer Ausführungsform der vorliegenden Erfindung umfasst eine Behälteraufnahme 3 zum Aufnehmen eines Behälters 5. In dem Behälter 5 ist eine Flüssigkeit 7 aufgenommen. Mittels der Vorrichtung 1 kann von der Probe 7 der Flammpunkt und/oder der Brennpunkt bestimmt werden. Die Vorrichtung 1 umfasst ferner einen Infrarot-Sensor 9, welcher angeordnet ist, Licht 11, das durch eine Flamme in einem Überwachungsbereich um oder innerhalb des Behälters 5 erzeugt ist, zu detektieren. Die Vorrichtung 1 umfasst ferner ein Auswertesystem 13, das mit dem Infrarot-Sensor 9 über eine Signalleitung 15 gekoppelt ist und ausgebildet ist, Infrarot-Sensor-Daten 17 zu erhalten und auszuwerten, um basierend auf der Auswertung einen Brand oder einen Burn anzuzeigen, wozu insbesondere ein Anzeigeschirm 19 vorgesehen ist. Der Infrarot-Sensor 9 umfasst ein intern eingebautes, nicht dargestelltes optisches Filter, das ausgebildet ist, auf einen für Wärmestrahlung empfindlichen Bereich selektiv zumindest einen Teil einer Absorptionsbande von Kohlendioxid auszufiltern, sowie ein Schutzfenster 10.

Die Vorrichtung 1 umfasst ferner einen Flammenionisationsdetektor 21 mit zwei Elektroden, einer nicht illustrierten Spannungsquelle und einer nicht illustrierten abgleichbaren Widerstands-Messschaltung, um Widerstands-Daten 23 zu messen, die einen Widerstand zwischen den Elektroden abhängig von einer Flammenionisation anzeigen. Die Widerstands-Daten werden dem Auswertesystem 13 über eine Datenleitung 25 zugeführt und das Auswertesystem ist ausgebildet, diese Widerstands-Daten 23 auszuwerten, wobei basierend auf einem Vergleich einer gemessenen Änderung des Widerstandes mit einem Schwellwert auf einem Brand oder Burn und/oder einer Gaszündflamme geschlossen wird.

Ein Teil 27 der Vorrichtung 1 ist als zweite Elektrode des Flammenionisationsdetektors 21 ausgeführt. Dieser Teil 27 der Vorrichtung bildet auch ein Gehäuse oder eine Ummantelung oder Halterung einer Zündvorrichtung 29 mit zumindest einer Zündspitze 31, 33, die zum Entzünden der Probe in den Behälter 5 hinein geradlinig entlang einer Richtung 35 verfahrbar ist. Das Teil 27 ist aus Metall gebildet und reicht am unteren zum Behälter 5 hin gerichteten Ende fast zu den Zündspitzen 31 und 33. Dabei gehört die Zündspitze 31 zu einem Gaszünder 37 und die Zündspitze 33 gehört zu einem Elektrozünder 39. Der Gaszünder 37 ist an die Ummantelung des Elektrozünders 39 aufgesteckt und ist abnehmbar. Die Zündvorrichtung 21 ist entlang von Richtungen, die durch den Doppelpfeil 35 angezeigt ist, nach oben und unten verfahrbar. Die zweite Elektrode des Flammenionisationsdetektors 21 kann auf ein Potential zwischen 1 V und 10 V gelegt werden.

Eine erste Elektrode des Flammenionisationsdetektors 21 ist durch einen Deckel 41 und/oder durch den Behälter 5 und/oder die Behälteraufnahme 3 und/oder die obere Fläche eines Basiskörpers 2 gebildet. Der Deckel und/oder der Behälter 5 und/oder die Behälteraufnahme 3 und/oder die obere Fläche des Basiskörpers 2 können auf Massepotential, d.h. Erdpotential gelegt werden. Der Flammenionisationsdetektor 21 umfasst ferner eine abgleichbare Widerstandsmessschaltung, um den Widerstand zwischen der ersten Elektrode 41 bzw. 5 und der zweiten Elektrode 27 zu messen. Der Widerstand zwischen den beiden Elektroden ändert sich in Abhängigkeit einer aufgrund einer Flammbildung erzeugten Ionisation der Moleküle zwischen den Elektroden.

Die Vorrichtung 1 umfasst den Basiskörper 2, in dem die Behälteraufnahme 3 angeordnet ist. Die Behälteraufnahme 3 ist mittels eines Temperier-Systems temperierbar. Insbesondere kann die Behälteraufnahme 3 als ein Temperierblock ausgebildet sein. Über ein Verbindungselement 4 (z.B. ein Gestänge oder ein Rohr) ist ein Gerätekopf 6 mit dem Basiskörper 2 verbunden. Dabei kann der Gerätekopf entlang der Richtung 8 relativ zum Basiskörper 2 nach oben und unten verfahren werden. Während einer Flammüberwachung kann der Infrarot-Sensor 9 in einem Bereich zwischen 5 cm und 30 cm von einer oberen Kante des Behälters 5 entfernt sein. Der Infrarot-Sensor empfängt Licht 11 aus einem gewissen Winkeleintrittsbereich 43, welcher derart gewählt ist, um einen gewünschten Überwachungsbereich abzudecken.

Die Vorrichtung 1 umfasst ferner ein Temperatur-Sensorsystem 45, das ausgebildet ist, den Flammpunkt der Probe (insbesondere mittels eines Temperatur-Messsensors 47) im gasförmigen Zustand und die Temperatur der Probe im flüssigen Zustand (mittels eines weiteren, nicht dargestellten Temperatur-Messsensors) innerhalb des Behälters 5 zu messen, um einen Flammpunkt und/oder einen Brennpunkt bestimmen zu können.

Der Behälter 5 ist über einen Griff 49 von einem Benutzer handhabbar, um den Behälter 5 aus der Behälteraufnahme 3 zu entnehmen. Der Temperatur-Messfühler 47 reicht durch eine Öffnung innerhalb des Behälterdeckels 41 in den Behälter 5 hinein. Bevor eine der Zündspitzen 31 bzw. 33 in das Innere des Behälters 5 entlang der Richtung 35 verfahren wird, wird der Behälterdeckel 41 durch einen auf diesem befindlichen Deckelschieber (nicht dargestellt) geöffnet. Die Vorrichtung 5 umfasst ferner eine Rührvorrichtung 51 mit einem Rührer 53, welcher mittels eines (nicht gezeigten) Elektromotors in eine Drehbewegung versetzt wird.

Die Vorrichtung 1 ist ausgebildet, ein Verfahren zur Flammüberwachung bei einer Flammpunktbestimmung und/oder Brennpunktbestimmung gemäß einer Ausführungsform der vorliegenden Erfindung durchzuführen.

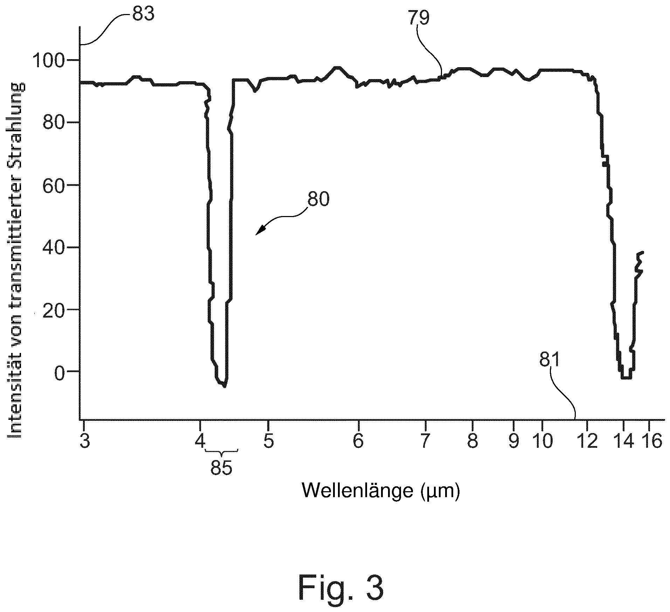

Ausführungsformen der vorliegenden Erfindung verwenden zur Flammüberwachung zwei Sensoren, die auf unterschiedlichen Brandüberwachungs-Prinzipien basieren, nämlich a) eine Flammenionisationsdetektion und b) eine optische Messung der CO₂-IR-Bande.
- Der Flammenionisationsdetektor (FID) 21 beruht auf dem Prinzip der Messung des Widerstandes der Luft. Tritt Verbrennung einer Substanz ein und eine Flamme entsteht, wird der Beobachtungsraum zwischen zwei Kondensatorplatten (hier Elektroden 27 einerseits und 41 oder 5 oder 3 oder die obere Fläche des Basiskörpers 2 andererseits) mit thermisch ionisiertem Dampf gefüllt. Die dabei freiwerdenden Elektronen und/oder Ionen werden durch die Kondensatorplatten aufgefangen und als Signal (z.B. 23) aufgezeichnet. Das Ansprechverhalten eines FIDs ist für die Überwachung eines Burns liegt im optimalen Bereich von einigen Millisekunden.
- Der zweite verwendete Sensor 9 beruht auf dem Prinzip von substanzspezifischen Infrarot(IR)-Transmission bzw. Absorptionsbande(n). Die bei einer Verbrennung entstehenden organischen Probenfragmente und Umwandlungsprodukte wie Wasser (H₂O) und Kohlenstoffdioxyd (CO₂) weisen im IR-Spektralbereich charakteristische Transmission- bzw. Absorptionsbanden auf. Im Wellenlängenbereich rund um 4.3 µm (Wellenzahl ~2350 cm⁻¹) liegt ein schmaler, stark ausgeprägter, CO₂-charakteristischer Peak, der gemäß Ausführungsformen zur Flammenüberwachung ausgewertet wird (siehe auch Fig. 3).

Über einen Gasschlauch 38 wird dem Gaszünder 37 ein entflammbares Gas zugeführt. Die Vorrichtung 1 kann ferner eine Messablaufsteuerung umfassen, um gemäß benutzerdefinierten oder standardisierten Tests eine Flammpunktbestimmung und/oder Brennpunktbestimmung der in dem Behälter 5 eingeführten Probe 7 durchführen zu können.

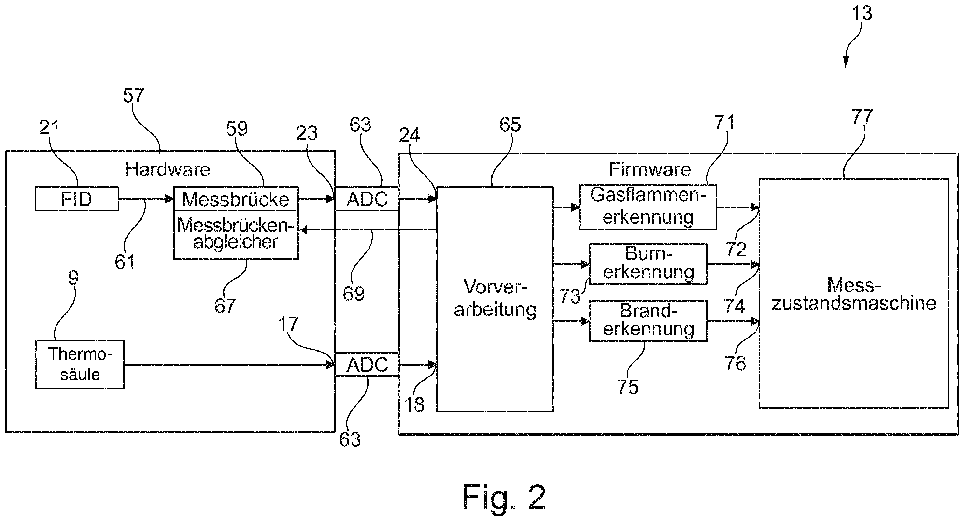

Fig. 2 illustriert im größeren Detail das in Fig. 1 illustrierte Auswertesystem 13 in schematischer Darstellung. Das Auswertesystem 13 ist mit dem Sensorsystem 57 gekoppelt, welches sowohl den Flammenionisationsdetektor 21 als auch den Infrarot-Detektor oder -Sensor 9, hier als "Thermopile" ausgeführt, umfasst. Über eine Messbrückenschaltung 59 werden Roh-Daten 61 des Flammenionisationsdetektors 21 in die Widerstands-Daten 23 überführt. Die Roh-Signale 61 des Flammenionisationsdetektors 21 werden dabei an die abgeglichene Messbrücke 59 angelegt, um Differenzen im Übergangs-Widerstand zwischen den beiden Elektroden 27, 41 bzw. 5 bzw. 3 bzw. die obere Fläche des Basiskörpers 2 zu messen. Ein Messbrücken-Abgleicher 67 ermöglicht, vor jeder Messung die Messbrücke 59 neu abzugleichen. Dazu wird der Messbrücken-Abgleicher 67 über eine Signalleitung 69 von der Vorverarbeitungseinheit 65 angesteuert. Das Signal von dem Infrarot-Sensor (z.B. Thermopile) 9 wird bereits von einer nicht illustrierten Elektronik vorverstärkt und als Signal 17 direkt als Analog-Werte an den Wandler 63 übergeben. Über den Analog-zu-Digital-Wandler 63 werden die analogen Widerstands-Daten 23 in digitale Widerstands-Daten 24 umgewandelt und der Vorverarbeitungseinheit 65 des Auswertesystems 13 zugeführt.

Die Infrarot-Sensor-Daten 17 werden ebenfalls über einen Analog-zu-Digital-Wandler 63 in entsprechende digitale Widerstands-Daten 18 überführt und der Vorverarbeitungseinheit 65 zugeführt.

Die Auswerteeinheit 13 umfasst ein Gasflammen-Erkennungsmodul 71, ein Burn-Erkennungsmodul 73 und ein Brand-Erkennungsmodul 75. Von dem Brand-Erkennungsmodul 75 und/oder Burn-Erkennungsmodul 73 werden sowohl die digitalen Widerstands-Daten 24 als auch die digitalen Infrarot-Sensor-Daten 18 herangezogen. Für die Gasflammenerkennung 71 werden (lediglich) die Widerstands-Daten 24 herangezogen. Insbesondere hat der Flammenionisationsdetektor 21 gemäß Ausführungsformen der vorliegenden Erfindung die Aufgaben der (1) Burn- und Brand-Erkennung und (2) Gasflammenüberwachung im Tiegeldeckel-Öffnungsbereich.
1. Burn-und Branderkennung: Der Burn kann eine Entflammung außerhalb des Tiegels umfassen. Er kann vorkommen, wenn der erwartete Flammpunkt falsch eingestellt wurde. Dafür eignet sich der FID besonders gut, da er auf Grund seiner schnellen Reaktionszeit von einigen Millisekunden ein sehr genaues Signal einer solchen Entflammung erkennen kann. Bei länger andauernder Flamme wird ein Brand signalisiert.
2. Gasflammenüberwachung: Der FID kann so konstruiert sein, dass er gleichzeitig als Schutzblech für die Elektro-und Gaszündung fungiert. Damit ist der FID sehr nahe an der Elektro- bzw. Gaszündung (31, 33). Wenn die Gasflamme durch einen Luftzug ausgeblasen wird, muss sie wieder entzündet werden. Ob die Gasflamme vorhanden ist, wird durch den FID 21 durch eine konstant höhere Ionisation der Luft festgestellt.

Die Einzelergebnisse 72, 74, 76 der Gasflammen-Erkennung 71, der Burn-Erkennung 73 und der Brand-Erkennung 75 werden einer Messzustandsmaschine 77 zugeführt. Im Falle einer Brand-Erkennung kann z.B. ein Löschverfahren eingeleitet werden, nachdem der normale Messprozess unterbrochen wurde. Im Falle einer Burn-Erkennung kann dem Benutzer dies angezeigt werden und er kann aufgefordert werden, die Konfigurations-Daten-Eingabe zu überprüfen (z.B. hinsichtlich eines erwarteten Flammpunktes oder Brennpunktes). Die Erkennung einer nicht vorhandenen Gasflamme kann dem Benutzer angezeigt werden, woraufhin die Gasflamme wieder entzündet werden kann, z.B. mittels der Elektrozünderspitze 33.

Der Thermopiledetektor 9 kann eine großflächige Brandüberwachung über den Messplatz durchführen und kann den FID bei der Entscheidung unterstützen, ob es sich um einen Burn handelt oder ob die Probe wasserhaltig ist. Bei der Entscheidung, ob es sich um einen Brand handelt, kann ein sogenanntes Voter-Prinzip (Abstimmungsprinzip) angewandt werden. Wenn beide Sensoren 9, 21 eine Warnung (oder ein Indiz) anzeigen, dass es sich um einen Brand handeln könnte, kann sofort (ein nicht dargestellter) Feuerlöschmechanismus aktiv werden. Wenn nur ein Sensor - entweder FID oder Thermopile - einen Brand erkennt, wird erst nach einer gewissen Zeitdauer, z.B. drei Sekunden, die Brandlöschung aktiv.

Fig. 3 illustriert ein Transmissionsspektrum 79 von Kohlendioxid im InfrarotBereich, wobei eine Abszisse 81 die Wellenlänge in Mikrometern anzeigt und eine Ordinate 83 die Intensität von durchgelassener Strahlung in Prozent anzeigt. In einem Wellenlängenbereich 85 zeigt das Transmissionsspektrum 79 eine Transmissionsbande 80 verringerter Transmission (was einer Absorptionsbande erhöhter Absorption entspricht), die gemäß Ausführungsformen der vorliegenden Erfindung mittels eines entsprechenden Bandpass-Filters selektiv auf einen Infrarot-Sensor hindurchgelassen wird, wobei Strahlung anderer Wellenlängen in ihrer Intensität wesentlich reduziert wird.

## Patentansprüche

1. Vorrichtung (1) zur Flammpunktbestimmung und/oder Brennpunktbestimmung einer in einem Behälter (5) aufnehmbaren flüssigen Probe (7) und zur Flammenerkennung, aufweisend :
eine Behälteraufnahme (3) zum Aufnehmen des Behälters (5);
einen Infrarot-Sensor (9), der angeordnet ist, Licht (11), das durch eine Flamme in einem Bereich um oder innerhalb des Behälters (5) erzeugt ist, zu detektieren;
einen Flammenionisationsdetektor (21) mit zwei Elektroden, einer Spannungsquelle, um an eine Elektrode eine elektrische Spannung anzulegen, und einer abgleichbaren Widerstandsmessschaltung (59), insbesondere Wheatstone'sche Messbrücke, um Widerstands-Daten (23) zu messen, die einen Widerstand zwischen den Elektroden abhängig von einer Flammenionisation anzeigen; und
ein Auswertesystem (13), das mit dem Infrarot-Sensor (9) gekoppelt ist und ausgebildet ist, Infrarot-Sensor-Daten (17, 18) des Infrarot-Sensors auszuwerten, um basierend auf der Auswertung einen Brand der Probe und/oder einen Burn der Probe anzuzeigen,
wobei ein Burn eine kurzzeitige Entflammung der Probe aufweist und ein Brand eine kontinuierliche Flamme aufweist.

2. Vorrichtung gemäß dem vorangehenden Anspruch, wobei der Infrarotsensor (9) einen für Wärmestrahlung empfindlichen Bereich sowie zumindest ein optisches Filter (10) aufweist, das ausgebildet und angeordnet ist, um von dem für Wärmestrahlung empfindlichen Bereich selektiv zumindest einen Teil zumindest einer Absorptionsbande (80) von Kohlendioxid zu detektieren.

3. Vorrichtung gemäß dem vorangehenden Anspruch, wobei die Absorptionsbande (80) in einem Wellenlängenbereich zwischen 4,2 mm und 4,4 mm liegt.

4. Vorrichtung gemäß einem der zwei vorangehenden Ansprüche, wobei der Infrarot-Sensor (9) ein oder mehrere Thermoelemente aufweist, die den für Wärmestrahlung empfindlichen Bereich definieren, und insbesondere als Thermosäule ausgebildet ist.

5. Vorrichtung gemäß einem der vorangehenden Ansprüche,
wobei das Auswertesystem (13) mit dem Flammenionisationsdetektor (21) gekoppelt ist und ausgebildet ist, die Widerstands-Daten (23, 24) auszuwerten, wobei basierend auf einem Vergleich einer gemessenen Änderung des Widerstandes mit einem Schwellwert auf einen Brand oder Burn und/oder eine Gaszündflamme geschlossen wird.

6. Vorrichtung gemäß einem der vorangehenden Ansprüche, wobei das Auswertesystem (13) ausgebildet ist:
eine Gasflammenerkennung (71) eines Gaszünders und/oder
eine Burnerkennung (73) und/oder
eine Branderkennung (75)
durchzuführen,
wobei für die Branderkennung und/oder die Burnerkennung sowohl die Widerstands-Daten als auch die Infrarot-Sensor-Daten herangezogen werden, wobei für die Gasflammenerkennung die Widerstands-Daten herangezogen werden.

7. Vorrichtung gemäß dem vorangehenden Anspruch,
wobei ein Brand erkannt wird, wenn sowohl die Widerstands-Daten (23, 24) als auch die Infrarot-Sensor-Daten (17, 18) eine Erhöhung über jeweilige Schwellwerte über einen Mindestzeitraum zeigen.

8. Vorrichtung gemäß einem der zwei vorangehenden Ansprüche, wobei ein Bum erkannt wird, wenn sowohl die Widerstands-Daten (23, 24) als auch die Infrarot-Sensor-Daten (17, 18) eine Erhöhung über jeweilige Schwellwerte für weniger als 1/10 des Mindestzeitraums zeigen.

9. Vorrichtung gemäß einem der vorangehenden Ansprüche, ferner aufweisend:
einen Behälter (5) zum Aufnehmen der flüssigen Probe (7) mit einer Behälteröffnung.

10. Vorrichtung gemäß dem vorangehenden Anspruch, wobei der Infrarotsensor (9) oberhalb der Behälteröffnung angeordnet ist, insbesondere in einem Bereich zwischen 5 cm und 30 cm von einer oberen Kante des Behälters entfernt.

11. Vorrichtung gemäß einem der zwei vorangehenden Ansprüche, wenn rückbezogen auf Anspruch 5, ferner aufweisend:
einen Behälterdeckel (41),
wobei eine erste der Elektroden durch den Behälter (5) und/oder den Behälterdeckel (41) gebildet ist und wobei die Spannungsquelle ausgebildet ist, die erste Elektrode auf Massepotential zu legen.

12. Vorrichtung gemäß dem vorangehenden Anspruch,
wobei eine zweite der Elektroden durch ein Metallteil (27), insbesondere in der Umgebung einer Zündvorrichtung (29), mit zumindest einer Zündspitze (31, 33) gebildet ist, die zum Entzünden der Probe in den Behälter hinein, insbesondere geradlinig, verfahrbar ist,
wobei die Spannungsquelle ausgebildet ist, die zweite Elektrode auf ein positives elektrisches Potential, insbesondere zwischen 1 V und 10 V, zu legen.

13. Vorrichtung gemäß dem vorangehenden Anspruch, wobei die Zündvorrichtung (29) einen Elektrozünder (39) und/oder einen, insbesondere, abnehmbaren Gaszünder (37) aufweist.

14. Vorrichtung gemäß einem der vorangehenden Ansprüche, ferner aufweisend:
ein Temperaturmesssystem (45), das ausgebildet ist, eine Temperatur der Probe im flüssigen und/oder gasförmigen Zustand innerhalb des Behälters zu messen, um einen Flammpunkt und/oder einen Brennpunkt zu bestimmen; und
ein Temperiersystem zum Temperieren der Probe.

15. Verfahren zur Flammüberwachung bei einer Flammpunktbestimmung und/oder Brennpunktbestimmung einer in einem Behälter (5) aufnehmbaren flüssigen Probe (7), aufweisend:
Aufnehmen des Behälters (5) in einer Behälteraufnahme (3);
Detektieren von Licht (11), das durch eine Flamme in einem Bereich um oder innerhalb des Behälters (5) erzeugt ist, mittels eines Infrarot-Sensors (9);
Anlegen, mittels einer Spannungsquelle, einer elektrischen Spannung zwischen zwei Elektroden eines Flammenionisationsdetektors (21) mit einer abgleichbaren Widerstandsmessschaltung (59);
Messen von Widerstands-Daten (23), die einen Widerstand zwischen den Elektroden abhängig von einer Flammenionisation anzeigen; und
Auswerten von Infrarot-Sensor-Daten (17, 18) des Infrarot-Sensors (9), um basierend auf der Auswertung einen Brand und/oder einen Burn anzuzeigen,
wobei ein Burn eine kurzzeitige Entflammung der Probe aufweist und ein Brand eine kontinuierliche Flamme aufweist.

## Claims

1. Device (1) for a flash point determination and/or a combustion point determination of a liquid sample (7) which is receivable in a container (5) and for a flame recognition, comprising:
a container reception (3) for receiving the container (5);
an infrared sensor (9) which is arranged for detecting light (11) which is generated by a flame in a region around or within the container (5);
a flame ionization detector (21) with two electrodes, a voltage source for applying an electric voltage to an electrode, and a calibratable resistance measuring circuit (59), in particular a Wheatstone measuring bridge, for measuring resistance data (23) which indicate a resistance between the electrodes depending on a flame ionization; and
an evaluation system (13) which is coupled with the infrared sensor (9) and which is configured for evaluating infrared sensor data (17, 18) of the infrared sensor, to indicate a combustion of the sample and/or a burn of the sample based on the evaluation,
wherein a burn comprises a short-time inflammation of the sample, and a combustion comprises a continuous flame.

2. Device according to the preceding claim, wherein the infrared sensor (9) comprises a region which is sensitive to heat radiation and at least one optical filter (10) which is configured and arranged for selectively detecting at least a part of at least one absorption band (80) of carbon dioxide from the region which is sensitive to heat radiation.

3. Device according to the preceding claim, wherein the absorption band (80) is in a wavelength range between 4.2 mm and 4.4 mm.

4. Device according to one of the two preceding claims, wherein the infrared sensor (9) comprises one or more thermal elements which define the region which is sensitive to heat radiation, and in particular is configured as a thermal column.

5. Device according to one of the preceding claims, wherein the evaluation system (13) is coupled with the flame ionization detector (21) and is configured for evaluating the resistance data (23, 24), wherein, based on a comparison of a measured change of the resistance with a threshold value, it is concluded to a combustion or a burn and/or a gas ignition flame.

6. Device according to one of the preceding claims, wherein the evaluation system (13) is configured for performing:
a gas flame recognition (71) of a gas igniter, and/or
a burn recognition (73), and/or
a combustion recognition (75),
wherein for the combustion recognition and/or the burn recognition both the resistance data and the infrared sensor data are used, wherein for the gas flame recognition, the resistance data are used.

7. Device according to the preceding claim, wherein a combustion is recognized, when both the resistance data (23, 24) and the infrared sensor data (17, 18) indicate an increase above respective threshold values over a minimum time interval.

8. Device according to one of the two preceding claims, wherein a burn is recognized, when both the resistance data (23, 24) and the infrared sensor data (17, 18) indicate an increase above respective threshold values for less than 1/10 of the minimum time interval.

9. Device according to one of the preceding claims, further comprising:
a container (5) for receiving the liquid sample (7) with a container opening.

10. Device according to the preceding claim, wherein the infrared sensor (9) is arranged above the container opening, in particular in a region between 5 cm and 30 cm spaced apart from an upper edge of the container.

11. Device according to one of the two preceding claims, when referring to claim 5, further comprising:
a container lid (41),
wherein a first one of the electrodes is formed through the container (5) and/or the container lid (41), and wherein the voltage source is configured for applying a ground potential to the first electrode.

12. Device according to the preceding claim, wherein the second one of the electrodes is formed through a metal part (27), in particular in the environment of an igniting device (29), with at least one igniting tip (31, 33) which is, in particular straightly, displaceable into the container for igniting the sample, wherein the voltage source is configured for applying a positive electric potential to the second electrode, in particular between 1 V and 10 V.

13. Device according to the preceding claim, wherein the igniting device (29) comprises an electric igniter (39) and/or a, in particular removable, gas igniter (37).

14. Device according to one of the preceding claims, further comprising:
a temperature measuring system (45) which is configured for measuring a temperature of the sample in a liquid and/or gaseous state within the container, to determine a flash point and/or a combustion point; and
a tempering system for tempering the sample.

15. Method for flame monitoring in a flash point determination and/or combustion point determination of a liquid sample (7) which is receivable in a container (5), comprising:
receiving the container (5) in a container reception (3);
detecting light (11) which is generated by a flame in a region around or within the container (5) by an infrared sensor (9);
applying, by a voltage source, an electric voltage between two electrodes of a flame ionization detector (21) with a calibratable resistance measuring circuit (59);
measuring resistance data (23) which indicate a resistance between the electrodes depending on a flame ionization; and
evaluating infrared sensor data (17, 18) of the infrared sensor (9) for indicating a combustion and/or a burn based on the evaluation,
wherein a burn comprises a short-time inflammation of the sample, and a combustion comprises a continuous flame.

## Revendications

1. Dispositif (1) pour la détermination du point éclair et/ou
la détermination du point de feu d'un échantillon liquide (7) qui peut être reçu dans un récipient (5) et pour la détection de flamme, comprenant :
un réceptacle de récipient (3) destiné à recevoir le récipient (5) ;
un capteur infrarouge (9) qui est agencé pour détecter la lumière (11) qui est générée par une flamme dans une région située autour ou à l'intérieur du récipient (5) ;
un détecteur à ionisation de flammes (21) avec deux électrodes, une source de tension pour appliquer une tension électrique à une électrode et un circuit de mesure de résistance réglable (59), en particulier un pont de mesure de Wheatstone, pour mesurer des données de résistance (23) qui indiquent une résistance entre les électrodes en fonction de l'ionisation de flammes ; et
un système d'évaluation (13) qui est couplé au capteur infrarouge (9) et qui est conçu pour évaluer des données de capteur infrarouge (17, 18) provenant du capteur infrarouge afin d'indiquer un feu de l'échantillon et/ou une combustion de l'échantillon sur la base de l'évaluation,
dans lequel une combustion présente une inflammation momentanée de l'échantillon et un feu présente une flamme continue.

2. Dispositif selon la revendication précédente, dans lequel le capteur infrarouge (9) présente une région sensible à un rayonnement thermique ainsi qu'au moins un filtre optique (10), qui est conçu et agencé pour détecter sélectivement au moins une partie d'au moins une bande d'absorption (80) de dioxyde de carbone à partir de la région sensible à un rayonnement thermique.

3. Dispositif selon la revendication précédente, dans lequel la bande d'absorption (80) se situe dans une plage de longueurs d'onde comprise entre 4,2 mm et 4,4 mm.

4. Dispositif selon l'une quelconque des deux revendications précédentes, dans lequel le capteur infrarouge (9) comprend un ou plusieurs thermocouples définissant la région sensible à un rayonnement thermique, et est notamment réalisé sous la forme d'une thermopile.

5. Dispositif selon l'une quelconque des revendications précédentes, dans lequel le système d'évaluation (13) est couplé au détecteur à ionisation de flammes (21) et est conçu pour analyser les données de résistance (23, 24),
dans lequel sur la base d'une comparaison d'une variation mesurée de la résistance avec une valeur de seuil sur un feu ou une combustion et/ou une flamme d'allumage de gaz.

6. Dispositif selon l'une quelconque des revendications précédentes, dans lequel le système d'évaluation (13) est réalisé pour effectuer :
une détection de flammes de gaz (71) d'un allumeur de gaz et/ou
une détection de combustion (73) et/ou
une détection de feu (75)
dans lequel les données de résistance et les données de capteur infrarouge sont utilisées pour la détection de feu et/ou la détection de combustion,
dans lequel les données de résistance sont utilisées pour la détection de flammes de gaz.

7. Dispositif selon la revendication précédente, dans lequel un feu est détecté si les données de résistance (23, 24) et les données de capteur infrarouge (17, 18) montrent une augmentation au-dessus de seuils respectifs sur une période minimale.

8. Dispositif selon l'une quelconque des deux revendications précédentes, dans lequel une combustion est détectée lorsque les données de résistance (23, 24) et les données de capteur infrarouge (17, 18) montrent une augmentation au-dessus des seuils respectifs pendant moins de 1/10 de la période minimale.

9. Dispositif selon l'une quelconque des revendications précédentes, comprenant en outre :
un récipient (5) pour recevoir l'échantillon liquide (7) avec une ouverture de récipient.

10. Dispositif selon la revendication précédente, dans lequel le capteur infrarouge (9) est situé au-dessus de l'ouverture de récipient, en particulier dans une zone comprise entre 5 cm et 30 cm à partir d'un bord supérieur du récipient.

11. Dispositif selon l'une quelconque des deux revendications précédentes, lorsqu'elle dépend de la revendication 5, comprenant en outre : un couvercle de récipient (41),
dans lequel une première des électrodes est formée par le récipient (5) et/ou le couvercle de récipient (41) et dans lequel la source de tension est conçue pour mettre la première électrode au potentiel de masse.

12. Dispositif selon la revendication précédente, dans lequel une seconde des électrodes est formée par une pièce métallique (27), en particulier au voisinage d'un dispositif d'allumage (29), avec au moins une pointe d'allumage (31, 33), qui peut être déplacée dans le récipient, en particulier en ligne droite, pour enflammer l'échantillon,
dans lequel la source de tension est conçue pour placer la seconde électrode à un potentiel électrique positif, en particulier entre 1 V et 10 V.

13. Dispositif selon la revendication précédente, dans lequel le dispositif d'allumage (29) comprend un allumeur électrique (39) et/ou un allumeur à gaz (37), en particulier amovible.

14. Dispositif selon l'une quelconque des revendications précédentes, comprenant en outre : un système de mesure de température (45) qui est conçu pour mesurer une température de l'échantillon à l'état liquide et/ou gazeux à l'intérieur du récipient afin de déterminer un point éclair et/ou un point de feu ; et un système de régulation de la température pour réguler la température de l'échantillon.

15. Procédé de surveillance de flamme lors de la détermination d'un point éclair et/ou de la détermination d'un point feu d'un échantillon liquide (7) pouvant être reçu dans un récipient (5), comprenant les étapes consistant à :
recevoir le récipient (5) dans un réceptacle de récipient (3) ;
détecter de la lumière (11) qui est générée par une flamme dans une région située autour ou à l'intérieur du récipient (5) au moyen d'un capteur infrarouge (9) ;
appliquer, au moyen d'une source de tension, une tension électrique entre deux électrodes d'un détecteur à ionisation de flammes (21) avec un circuit de mesure de résistance réglable (59) ;
mesurer des données de résistance (23) indiquant une résistance entre les électrodes en fonction de l'ionisation de flammes ; et
évaluer des données de capteur infrarouge (17, 18) du capteur infrarouge (9) pour indiquer un feu et/ou une combustion sur la base de l'évaluation,
dans lequel une combustion présente une inflammation momentanée de l'échantillon et un feu présente une flamme continue.
